(19) Europäisches Patentamt

European Patent Office

Office européen des brevets

(11) Veröffentlichungsnummer: **0 291 060 B1**

(12) **EUROPÄISCHE PATENTSCHRIFT**

(45) Veröffentlichungstag der Patentschrift: **27.01.93**

(21) Anmeldenummer: **88107646.7**

(22) Anmeldetag: **11.05.88**

(51) Int. Cl.⁵: **C12Q 1/00**, C12Q 1/28, C12Q 1/26

(54) **Verfahren zur Bestimmung von Fructosamin.**

(30) Priorität: **14.05.87 DE 3716218**
**21.12.87 DE 3743405**

(43) Veröffentlichungstag der Anmeldung:
**17.11.88 Patentblatt 88/46**

(45) Bekanntmachung des Hinweises auf die
Patenterteilung:
**27.01.93 Patentblatt 93/04**

(84) Benannte Vertragsstaaten:
**AT BE CH DE ES FR GB GR IT LI LU NL SE**

(56) Entgegenhaltungen:
EP-A- 0 004 857      EP-A- 0 085 263
EP-A- 0 215 170      EP-A- 0 250 991
DE-A- 2 910 737      DE-A- 3 329 952

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Vogt, Bernd, Dr.**
**Mozartstr. 1**
**W-8132 Tutzing(DE)**
Erfinder: **Schellong, Lieselotte**
**Hallberger Allee 8**
**W-8132 Tutzing(DE)**
Erfinder: **Siedel, Joachim, Dr.**
**Weilheimer Str. 10**
**W-8139 Bernried(DE)**
Erfinder: **Ziegenhorn, Joachim, Dr.**
**Ina Seidel-Weg 1**
**W-8130 Starnberg(DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86(DE)**

**Beschreibung**

Die vorliegende Erfindung betrifft ein Verfahren zur Bestimmung von Fructosamin in Körperflüssigkeiten durch Umsetzung der Probenlösung mit einem Farbreagenz und mit oxidierend wirkenden Enzymen, sowie ein dazu geeignetes Reagenz.

Fructosamine bilden sich im Blut aus der vorhandenen Glucose. Die Carbonylgruppe der Glucose reagiert dabei mit freien Proteinamino-Resten unter Bildung von Schiff'schen Basen. Durch Amadori-Umlagerung entstehen dann die Fructosamine, die eine stabile Ketoaminbindung aufweisen. Die Halbwertszeit des Fructosamins ist wegen der Stabilität dieser Ketoaminbindung praktisch identisch mit der der Serumproteine, deren Halbwertszeit im Durchschnitt ca. 21 Tage beträgt. Da das Ausmaß der Fructosaminbildung proportional ist dem Blutglucosespiegel, gibt der Fructosamingehalt Aufschluß über den Zuckerstoffwechsel. Der Zuckerstoffwechsel muß beispielsweise bei Diabetikern ständig überwacht werden. Da der Blutglucosespiegel starken Schwankungen unterliegt, gibt er dem Arzt nur Auskunft über die zum Zeitpunkt der Blutentnahme vorhandene Stoffwechsellage. Eine weitere Möglichkeit zur Überprüfung des Zuckerstoffwechsels besteht in der Bestimmung des glycosylierten Hämoglobins ($HbA_1$). Diese Bestimmung ist für eine Langfristkontrolle des Zuckerstoffwechsels geeignet. Um nun auch eine mittelfristige Kontrolle zu haben, ist die Messung des Fructosamingehaltes sehr gut geeignet, da dessen Halbwertszeit die Stoffwechselführung von Diabetikern durch Lebenshaltung und therapeutische Maßnahmen über einen mittelfristigen Zeitraum von ca. 3 Wochen erlaubt. In Verbindung mit den bekannten klinischdiagnostischen Parametern Blutglucose, sowie glycosyliertes Hämoglobin kann daher über die Serumfructosamin-Bestimmung eine weitere zuverlässige spezifische und praktikable Methode zur Überwachung von Diabetikern zur Verfügung gestellt werden.

Die bisher bekannten Verfahren zur Bestimmung des Fructosamins (z.B. Johnson et al., Clin.Chim.Acta (1982) 127, 87-95) beruhen darauf, daß das Fructosamin, das in wäßrigem alkalischem Milieu in Enolform vorliegt und in dieser Form leicht oxidiert werden kann, umgesetzt wird mit einem Oxidationsmittel (farbgebende Verbindung), das in reduzierter Form farbig ist, beispielsweise Tetrazoliumsalze. Der dabei gebildete Formazanfarbstoff kann dann photometrisch gemessen werden und ist der Menge an Fructosamin proportional. Ein derartiges Verfahren wird beispielsweise auch in der EP-A 0 085 263 beschrieben.

Dieses Testprinzip hat jedoch den Nachteil, daß neben Fructosamin alle im Probenmaterial vorliegenden leicht oxidierbaren Bestandteile, wie beispielsweise Harnsäure und Bilirubin oder Medikamente wie α-Methyldopa und Medikamentabbauprodukte sowie auch Ascorbinsäure, zu verfälschten Meßergebnissen führen, da sie ebenfalls die farbgebenden Verbindungen reduzieren und damit zu einer zusätzlichen Farbstoffbildung führen.

Weiterhin werden die bekannten Fructosamin-Bestimmungsverfahren durch den von Probe zu Probe variierenden Gesamtproteingehalt gestört. Dies führt zu Meßwertschwankungen und verringert dadurch die Empfindlichkeit des Bestimmungsverfahrens. Diese Störungen sind als Matrixeffekte bekannt und treten besonders dann auf, wenn zusätzliche Proteine zugegeben werden, wie dies z.B. bei der Herstellung von Standardlösungen üblicherweise der Fall ist. Beispielsweise verlangsamen steigende Proteinmengen die Farbreaktion mit Desoxymorpholinofructose (DMF), welches häufig als Fructosaminanalogon in solchen Standardlösungen für Eichzwecke eingesetzt wird.

Schließlich hat ein Vergleich mit einer HPLC-Bezugsmethode (E. Schleicher et al., J.Clin.Chem.Clin.Biochem. 19 (1981) 81-87) gezeigt, daß mit dem bekannten Verfahren ein hoher Farbsignal-Achsenabschnitt (entsprechend ca. 50 % des Signals eines durchschnittlichen Normalserumkollektivs) erhalten wird.

Weitere Schwierigkeiten treten bei der Fructosamin-Bestimmung in hyperlipämischen Seren auf. Im allgemeinen ist, um ein auch bei niedrigen Fructosamin-Konzentrationen in der Probe noch ausreichend großes Meßsignal zu erhalten, ein Verhältnis von Probe zu Reagenz von 0,1 erforderlich. Bei überhöhten Triglyceridkonzentrationen macht sich jedoch bei einem so hohen Probenanteil die resultierende Trübung des Testansatzes negativ bei der photometrischen Messung bemerkbar. Die Fructosamin-Bestimmung wird dann erheblich erschwert oder sogar verhindert.

Die EP-A 0 004 857 offenbart die Verwendung von speziellen Reaktionsgemischen niedermolekularer organischer Verbindungen und mit diesen einen weniger löslichen Komplex bildenden Detergenzien zur Entfernung von Trübungen aus biologischem Probenmaterial. Die beschriebene Verfahrensführung ist jedoch kompliziert und die Beseitigung von Störungen durch ikterische und harnstoffreiche Seren unmöglich.

In der EP-A 0 215 170 wird eine Fructosaminbestimmung unter Verwendung von Tetrazoliumsalzen bei pH 10 bis 14 als Endproduktbestimmung beschrieben. Störungen durch Ascorbinsäure und Glutathion werden hierbei durch Zugabe von starken Basen, Oxidationsmitteln, Enzymen oder durch Aussalzen beseitigt. Damit können aber die Störungen durch lipämische, ikterische und harnsäurereiche Seren nicht beseitigt werden.

Außerdem erfordert die Testführung nach EP-A 0 215 170 eine zeitaufwendige und in die meisten Analysenautomaten nicht integrierbare Vorreaktion.

Einige dieser Schwierigkeiten wurden nun gelöst durch ein zweistufiges Verfahren, bei dem in einer ersten Stufe in der Probenlösung ein neutraler bis saurer pH-Wert eingestellt wird, bei dem das Fructosamin in der Ketoform vorliegt und daher praktisch nicht oxidiert werden kann. Bei diesem pH-Wert werden dann oxidierend wirkende Enzyme zugesetzt, bis die unspezifisch reduzierend wirkenden Probenbestandteile abreagiert haben. Sodann wird der pH-Wert erhöht in den alkalischen Bereich, wodurch das Fructosamin wiederum in seine Enolform übergeht und dann das Tetrazoliumsalz zugegeben, das mit dem Fructosamin reagiert. Auf diese Weise konnte die Genauigkeit und die Empfindlichkeit der bekannten Fructosamin-Verfahren sehr verbessert werden.

Aufgabe der vorliegenden Erfindung war es daher, ein Verfahren zu schaffen, das in einer Stufe durchgeführt werden kann und eine mindestens ebenso genaue und empfindliche Bestimmung des Fructosamins in Körperflüssigkeiten gestattet, wie sie bereits für das zweistufige Verfahren erreicht wird.

Diese Aufgabe wird gelöst durch ein Verfahren zur Bestimmung von Fructosamin in Körperflüssigkeiten durch Umsetzung der Probenlösung mit einem Farbreagenz, das dadurch gekennzeichnet ist, daß man die Probenflüssigkeit mit einer Pufferlösung mit einem pH-Wert von 9 bis 12, einer farbgebenden Verbindung, Uricase, sowie mindestens einem Detergens versetzt und die zeitliche Änderung der Extinktion in einem Temperaturbereich zwischen 20 und 40°C frühestens nach 5 Minuten kinetisch mißt.

Überraschenderweise wurde festgestellt, daß bei Zugabe der Komponenten farbgebende Verbindung, Uricase und Detergens zu der Probenlösung bei einem pH-Wert von 9 bis 12 die störenden oxidierbaren Verbindungen rasch abreagieren sowie Trübungen rasch aufgehellt werden, so daß nach einer kurzen Vorinkubationszeit spezifisch nur noch Fructosamin mit der farbgebenden Verbindung reagiert. Auf diese Weise ist es möglich, in einem einstufigen Verfahren das Fructosamin mit größerer Genauigkeit zu bestimmen, als es mit den bisher bekannten Verfahren möglich war.

Zur Bestimmung des Fructosamins wird die Probenflüssigkeit mit einer Pufferlösung auf einen pH-Wert von 9 bis 12 eingestellt. Bei diesem alkalischen pH-Wert liegt das Fructosamin dann in seiner Enolform vor. Bevorzugt wird der pH-Wert in einem Bereich von 10 bis 11 gehalten. Zur Herstellung der Pufferlösung sind alle Substanzen geeignet, die selbst nicht reduzierend wirken und deren Pufferwirkung ungefähr im gewünschten Bereich liegt. Besonders bevorzugt wird Carbonatpuffer verwendet, dessen Konzentration bevorzugt im Bereich von 50 bis 500 mmol/l liegt.

Um störende Probenbestandteile abreagieren zu lassen, wird Uricase zugesetzt. Mit diesem Enzym werden die hauptsächlich störenden Substanzen wie z. B. Harnsäure oxidiert und können die Bestimmung nicht stören. Die Konzentration der Uricase richtet sich dabei nach der Konzentration der zu beseitigenden störenden Verbindungen.

Üblicherweise liegen diese Konzentrationen in einem Bereich zwischen 1 bis 15 U/ml. So wird beispielsweise die Uricase bevorzugt in einer Konzentration von 2 bis 10 U/ml eingesetzt.

Vorzugsweise wird ein peroxidatisch wirksames Enzym zugesetzt. Dadurch kann eine weitere Verbesserung, insbesondere bezüglich der Korrelationder HPLC-Bezugsmethode erreicht werden.

Das peroxidatisch wirksame Enzym liegt bevorzugt in einer Konzentration von 1 bis 5 U/ml vor.

Als peroxidatisch wirksame Enzyme sind Verbindungen geeignet, die unter $H_2O_2$-Verbrauch oxidierend wirken wie z. B. Peroxidase (EC 1.11.1.7) oder Mikroperoxidasen (häminhaltige niedermolekulare Spaltprodukte der Peroxidase). Bevorzugt wird Peroxidase, insbesondere aus Meerrettich, verwendet. Besonders bevorzugt wird eine an ein wasserlösliches Polymeres gebundene Peroxidase, wie sie in DE 35 41 186.4 beschrieben ist, verwendet.

Als weitere Komponente wird der Probenflüssigkeit mindestens ein Detergens zugesetzt. Detergenzien dienen in erster Linie dazu, trübungsverursachende Probenbestandteile zu beseitigen. Darüberhinaus eliminieren sie überraschenderweise auch den Einfluß der Proteinmatrix und verbessern damit die Linearität der Beziehung zwischen Meßsignal und Konzentration des Probenproteins. Hierfür steht eine breite Palette von Detergentien zur Auswahl, die alle geeignet sind. Bevorzugt wird als Detergens ein nichtionisches, anionisches und/oder zwitterionisches Detergens verwendet. Besonders bevorzugt ist ein Gemisch aus einem nichtionischen und anionischen Detergens. Als nichtionisches Detergens wird dabei bevorzugt ein gerad- oder verzweigtkettiger Alkanol-Polyglykolether mit ca. 8 bis 12 C-Atomen im Alkanolteil und durchschnittlich 4 bis 8 Glykoleinheiten pro Molekül verwendet. Ein bevorzugtes anionisches Detergenz ist ein Alkalisalz einer Gallensäure, wobei Natriumcholat besonders bevorzugt eingesetzt wird. Ein bevorzugtes zwitterionisches Detergens ist ein Gallensäurederivat wie 3((3-Cholamidopropyl)dimethylammonio)-1-propansulfonat (CHAPS). Besonders bevorzugt wird außerdem noch ein kationisches Detergens zugesetzt, so daß in der Probenlösung mehrere verschiedene Detergenzien vorliegen. Als kationisches Detergens werden bevorzugt quartäre Ammoniumverbindungen oder quartäre Pyridiniumverbindungen oder Mischungen dieser beiden Substanzklassen verwendet. Die Konzentration der Detergenzien kann in weiten Bereichen variiert werden. Als geeignet haben sich Konzentrationen im Bereich von 0,5 bis 10 Gew.-%, bezogen auf den Reinsubstanzgehalt, erwiesen. Das nichtionische Detergens wird besonders bevorzugt in einer Konzentration von 1 bis 5 Gew.-% eingesetzt. Das anionische bzw. zwitterionische Detergenz wird besonders bevorzugt in einem Bereich von 1 bis 10 mmol/l verwendet. Das kationische Detergenz kann bevorzugt in einer Konzentration bis 5 Gew.-% verwendet werden.

Als farbgebende Verbindungen sind Verbindungen geeignet, die auf das Fructosamin oxidierend wirken und dabei ihre Farbe ändern wie z. B. Tetrazoliumsalzverbindungen. Tetrazoliumsalze sind in der analytischen Chemie bekannt und werden häufig verwendet. Sie sind Derivate des 1,2,3,4-Tetrazols und weisen einen quartären Stickstoff auf. Tetrazoliumsalze werden leicht reduziert und bilden unter Wasserstoff-Anlagerung die stark gefärbten Formazane, die photometrisch sehr gut bestimmt werden können. Es gibt eine Vielzahl von Tetrazoliumsalzen, die für das erfindungsgemäße Verfahren geeignet sind. Beispiele hierfür sind 3-(4,5-dimethylthiazolyl-2-)-2,4-diphenyl-tetrazoliumbromid (MTT), 2-(p-Iodophenyl)-3-(p-nitrophenyl)- 5-phenyltetrazoliumchlorid (INT), 2.2$'$,5.5$'$-Tetra-(p-nitrophenyl)-3.3$'$(3.3$'$-dimethoxy-4.4$'$-diphenylen)-ditetrazoliumchlorid (TNBT), 2.2$'$-Di(p-nitrophenyl)-5.5$'$-diphenyl-3.3$'$-(3.3$'$-dimethoxy,4.4$'$-diphenylen)-ditetrazoliumchlorid (NBT), 2.2$'$-p-Diphenylen-3.3$'$,5.5$'$-tetraphenyl-ditetrazoliumchlorid (Neotetrazoliumchlorid) (NT), 2.3.5-Triphenyltetrazoliumchlorid (TT). Besonders bevorzugt wird als Tetrazoliumsalz das auch Nitrotetrazoliumblau genannte NBT verwendet.

Die farbgebenden Verbindungen werden bevorzugt in einer Konzentration von 0,1 bis 1 mmol/l, besonders bevorzugt 0,2 bis 0,6 mmol/l verwendet.

Zur Bestimmung des Fructosamins in der Probenflüssigkeit werden alle Komponenten in einer Stufe zugegeben. Die Umsetzung erfolgt bei Temperaturen im Bereich von etwa 20 bis 40°C. Bevorzugt wird die Inkubation bei Temperaturen im Bereich von 25 bis 37°C durchgeführt.

Zur Bestimmung des Fructosamins wird die Reaktion photometrisch verfolgt. Gleich nach Zugabe aller Komponenten reagieren niedermolekulare Serumbestandteile sehr schnell ab und es kommt zu einem steilen Anstieg der Extinktionskurve. Nach Abreaktion der störenden Substanzen reagiert nur noch das Fructosamin und die Extinktionskurve flacht sich etwas ab. In diesem Bereich sollte dann die kinetische Messung erfolgen. Dies ist etwa 5 bis 15 Minuten nach Zugabe der Reagenzien der Fall. In diesem Zeitintervall werden dann mindestens 2 Messungen durchgeführt. Die zeitlichen Abstände zwischen den Messungen sind variabel, sie können abhängig von der apparativen Ausstattung ausgewählt werden. Der zeitliche Abstand kann im Bereich weniger Sekunden, aber auch einiger Minuten liegen.

In manchen Fällen ist es zweckmäßig, die erhaltenen Meßwerten mit denen einer Standardlösung zu vergleichen. Geeignete Standardlösungen sind bekannt. Beispielsweise kann hierzu der von Johnson et al. in Clin. Chim. Acta (1982) 127, 87-95 beschriebene Standard verwendet werden, der auf einer Matrix aus Humanalbumin mit definierten Zusätzen eines synthetischen Fructosamins basiert. Als synthetisches Fructosamin wird 1-Desoxy-1-morpholino-fructose (DMF) verwendet. Die ermittelte Serumfructosamin-Konzentration in der Probe wird bei Verwendung dieses Standards in DMF-Äquivalenten angegeben.

Überraschenderweise ist es mit dem erfindungsgemäßen Verfahren auch möglich, das Verhältnis von Probe zu Reagenzvolumen von 0,1, das nach dem Verfahren von Johnson et al. üblich ist, stark herabzusetzen, ohne daß bei gleichem Meßintervall und Inkubation bei gleicher Temperatur das Meßsignal mit einer definierten Menge an einem als Probe eingesetzten Fructosaminalogon signifikant im Vergleich zu der von Johnson et al. beschriebenen Methode kleiner wird. Gleichzeitig wird der Proteinmatrixeinfluß linearisiert. Zusätzlich wird praktisch kein Achsenabschnitt bei einem Vergleich mit der bereits erwähnten HPLC-Bezugsmethode beobachtet.

Das erfindungsgemäße Verfahren zur Bestimmung von Fructosamin in Körperflüssigkeiten läßt sich sowohl in Lösung als auch auf einem trockenchemischen Testträger durchführen. Es können die erfindungsgemäßen Komponenten in bekannter Weise auf feste Träger aufgebracht werden. Geeignete feste Träger, sowie Verfahren zum Aufbringen dieser Stoffe bzw. Stoffgemische auf solche Träger sind dem Fachmann bekannt. Als Trägermaterialien eignen sich beispielsweise saugfähige Materialien wie Papiere und Vliese. Die aufzubringenden Stoffe können in einer oder mehreren Imprägnierlösungen aufgenommen werden. Mit diesen Lösungen werden die Träger imprägniert oder besprüht und anschließend getrocknet. Eine andere Variante besteht darin, die erfindungsgemäß verwendeten Komponenten in Reagenzfilme einzubringen. Hierzu werden die Substanzen bzw. Substanzgemische z.B. entsprechend Verfahren gemäß DE-PS 1598153 oder DE-OS 2910134 zu Reagenzfilmen verarbeitet.

Ein weiterer Gegenstand der Erfindung ist ein Reagenzgemisch zur Bestimmung von Fructosamin in Körperflüssigkeiten enthaltend ein Farbreagenz, das dadurch gekennzeichnet ist, daß es eine Puffersubstanz mit einem pH-Wert von 9 bis 12, eine farbgebende Verbindung, Uricase, sowie mindestens ein Detergens enthält. Das Reagenzgemisch kann in Lösung vorliegen, es kann aber auch in an sich bekannter Weise in getrockneter Form oder auf einem Träger imprägniert vorliegen. Vorzugsweise wird zusätzlich ein peroxidatisch wirksames Enzym zugesetzt.

Bevorzugt wird als farbgebende Verbindung ein Tetrazoliumsalz verwendet.

Die Erfindung gestattet, das Fructosamin in einfacher Weise sehr genau und spezifisch zu bestimmen. Überraschenderweise gelingt dies durch eine Kombination mehrerer Komponenten.

Die Erfindung wird noch durch die folgenden Beispiele und die Figuren erläutert.

Fig. 1 Vergleich des erfindungsgemäßen Verfahrens bei Durchführung am Analysenautomaten mit der HPLC-Bezugsmethode. Aufgetragen sind die nach dem erfindungsgemäßen Verfahren ermittelten Fructosaminkonzentrationen von Patientenseren gegen relative Einheiten die mit der HPLC-Methode erhalten werden. (Korrelationskoeffizient: 0,965).

Fig. 2 Vergleich des Verfahrens nach Johnson et al. mit der HPLC-Methode (Korrelationskoeffizient: 0,933).

Fig. 3 Vergleich des erfindungsgemäßen Verfahrens bei manueller Durchführung mit der HPLC-Methode. (Korrelationskoeffizient: 0,995).

**Beispiel 1**

Bestimmung von Fructosamin im Serum nach dem erfindungsgemäßen Verfahren am Analysenautomaten.

Reagenzzusammensetzung:

200 mmol/l Natriumcarbonatpuffer pH 10,35, 1,2 Gew.-% nichtionisches Detergenz (Lutensol® ON 60), 3,5 mmol/l Natriumcholat, 4 U/ml Uricase, 2 U/ml Peroxidase, 0,5 mmol/l Nitrotetrazoliumblau (NBT).

Zu 350 $\mu$l Reagenz werden 7 $\mu$l Probe zugegeben und bei 37°C an einem Hitachi 704 Analysenautomaten bei $\lambda$ = 546/700 nm die Extinktion bestimmt. Die Kinetik wurde zwischen der 8. und 10. Minute nach Zugabe des Reagenzes ausgewertet.

Diese Ergebnisse wurden gegen die Meßwerte aufgetragen, welche mit der HPLC-Bezugsmethode (J. Clin. Chem., Clin. Biochem. 19, (1981, 81-87) erhalten wurden. Als HPLC-Meßwerte werden jeweils relative Peakflächeneinheiten verwendet.

Auch bei den nachfolgenden Beispielen wird die HPLC-Methode als Referenzmethode verwendet.

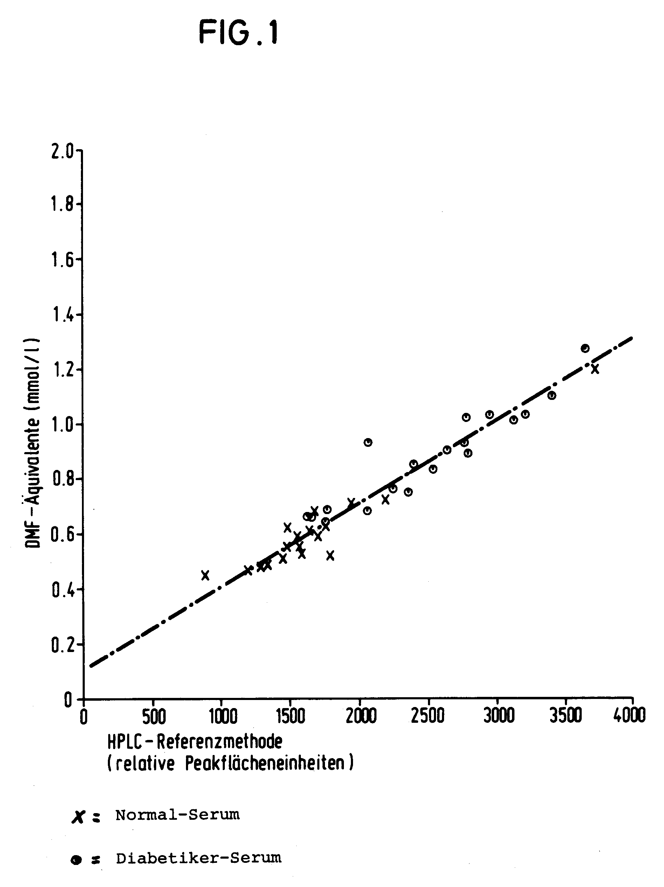

Das Ergebnis zeigt Fig. 1.

**Beispiel 2**

Bestimmung von Fructosamin im Serum nach der Methode von Johnson et al. am Analysenautomaten.

Reagenzzusammensetzung:

0,1 mol/l Natriumcarbonatpuffer pH 10,35
0,25 mmol/l Nitrotetrazoliumblau

Durchführung der Bestimmung:

20 $\mu$l Probe werden mit 200 $\mu$l Reagenz und 50 $\mu$l $H_2O$ (Diluent) vermischt und bei 37°C an einem Cobas Bio® Analysenautomaten (Hoffmann LaRoche) bei $\lambda$ = 530 nm die Extinktion zeitabhängig bestimmt. Zur Ermittlung der Fructosaminkonzentration wird die Kinetik zwischen der 10. und 15. Minute nach Zugabe des Reagenzes ausgewertet.

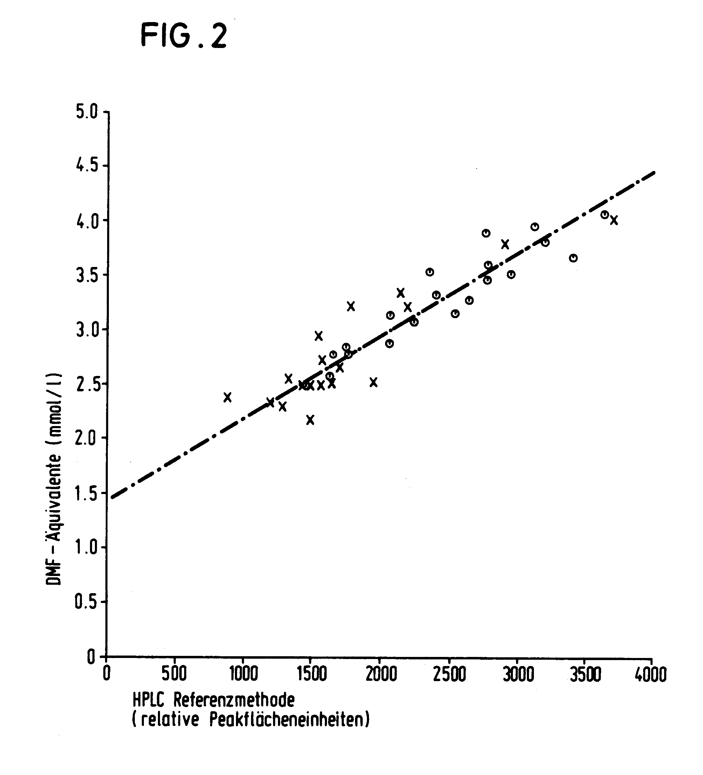

Das Ergebnis ist in Fig. 2 dargestellt.

Ein Vergleich der Ergebnisse von Beispiel 1 und Beispiel 2 zeigt, daß mit dem erfindungsgemäßen Verfahren gegenüber dem Stand der Technik eine Verbesserung der Korrelation der Meßwerte und eine wesentliche Verminderung des Achsenabschnitts beim Vergleich mit der HPLC-Referenzmethode erhalten wird.

**Beispiel 3**

Bestimmung von Fructosamin im Serum nach dem erfindungsgemäßen Verfahren bei manueller Testführung.

Die Reagenzzusammensetzung ist analog den Angaben von Beispiel 1.

Zu 20 $\mu$l Probe werden 1 ml Reagenz bei 37°C zugegeben und die Kinetik im Bereich zwischen der 10. und 15. Minute nach Probenzugabe, bei 546 nm gemessen, ausgewertet.

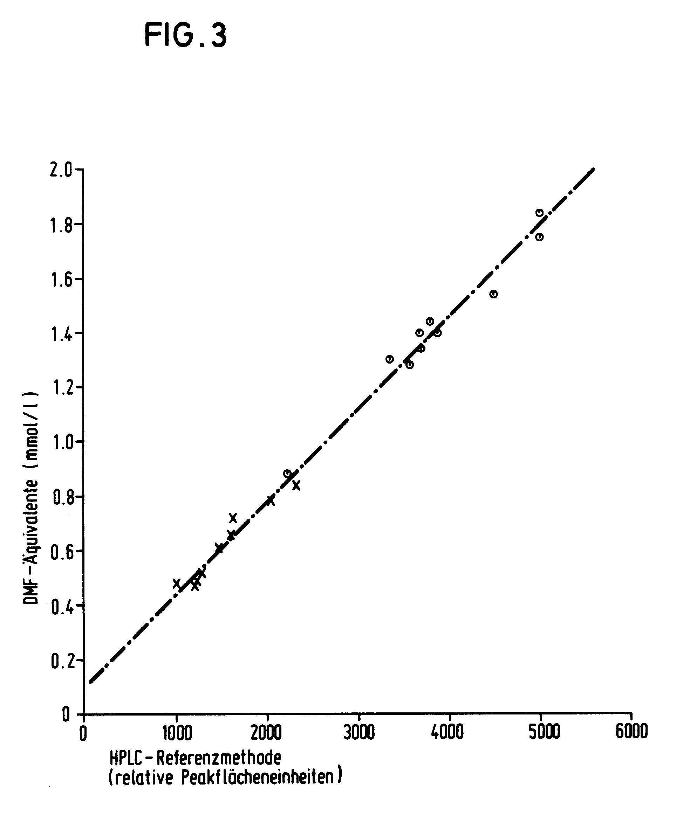

Wird die Messung bei Raumtemperatur durchgeführt, so muß aufgrund der geringeren Reaktivität die Auswertung über einen längeren Zeitraum durchgeführt werden (zwischen der 10. und 30. Minute). Die Ergebnisse sind analog (Fig. 3).

**Beispiel 4**

Vergleich der Störung der Fructosaminbestimmung durch lipämische Seren, Bilirubin und Harnsäure zwischen erfindungsgemäßem Verfahren und Verfahren nach Johnson et al.

Es werden die in Beispiel 1 und 2 beschriebenen Reagenzien und Verfahrensweisen verwendet.

Die Auswirkung der verschiedenen Störfaktoren auf beide Methoden ergibt sich aus Tabelle I. Dort ist die mittlere Abweichung von der Regressionsgeraden nach Fig. 1 und 2 angegeben. Dabei zeigt sich, daß der Einfluß aller Störfaktoren bei dem erfindungsgemäßen Verfahren geringer ist als nach dem Stand der Technik.

T a b e l l e   I

| Störung | mittlere Abweichung von der Regressionsgeraden | |
| --- | --- | --- |
| | Beispiel 1 (gemäß Erfindung) | Beispiel 2 (gemäß Johnson et al.) |
| lipämische Seren (n=8) mittlerer Triglyce- ridgehalt 1440 mg/dl (600 - 2280 mg/dl) | + 7 % | - 25 % |
| ikterische Seren (n=9) mittlerer Bilirubin- gehalt 16,7 mg/dl (8,2 - 32 mg/dl) | + 22 % | + 46 % |
| Harnsäure-Seren (n=10) mittlerer Harnsäure- gehalt 9,5 mg/dl (8,2 - 13,4 mg/dl) | - 2 % | - 7 % |

**Beispiel 5**

Eliminierung des Proteinmatrixeinflusses durch Detergenzzusatz.

Es wird analog Beispiel 1 gearbeitet. Als "Reagenz ohne Detergenz" wird ein Reagenz entsprechend Beispiel 1 verwendet, welches kein Natriumcholat und kein Lutensol enthält. Als Probe wird Desoxymorpholino-Fructose (DMF) in einer Konzentration von 2,5 mMol/l verwendet. Als Protein wird Rinderserumalbumin (RSA) verwendet. Die Ergebnisse zeigt Tabelle II.

T a b e l l e    II

| Proteinmatrix g/dl RSA | DMF-Signal (mE/min) | |
|---|---|---|
| | Reagenz gemäß Beispiel 1 | Reagenz ohne Detergenz |
| 2 | 16 | 12 |
| 4 | 15 | 11 |
| 6 | 16 | 10 |
| 8 | 15 | 9 |
| 10 | 15 | 8 |

Das DMF-Signal (Extinktion) ergibt sich aus der Differenz des Meßwertes für DMF + RSA, von dem der Meßwert für RSA allein abgezogen wird.

Es zeigt sich, daß das DMF-Signal bei Verwendung eines Reagenzes gemäß Beispiel 1 von der Proteinmatrix unabhängig ist, dagegen im sonst gleichen Reagenz ohne Detergenzzusatz mit zunehmendem Proteingehalt abnimmt.

**Patentansprüche**

1. Verfahren zur Bestimmung von Fructosamin in Körperflüssigkeiten durch Umsetzung der Probenlösung mit einem Farbreagenz und oxidierend wirkenden Enzymen,
**dadurch gekennzeichnet,**
daß man die Probenflüssigkeit mit einer nicht reduzierend wirkenden Pufferlösung mit einem pH-Wert von 9 bis 12, einer Tetrazoliumsalzverbindung und Uricase, sowie mindestens einem Detergens versetzt und die zeitliche Änderung der Extinktion in einem Temperaturbereich zwischen 20 und 40°C nach einer Vorinkubationszeit von wenigstens 5 Minuten kinetisch mißt.

2. Verfahren nach Anspruch 1,
**dadurch gekennzeichnet,**
daß man ein peroxidatisch wirksames Enzym zusetzt.

3. Verfahren nach Anspruch 1 oder 2,
**dadurch gekennzeichnet,**
daß eine Pufferlösung mit einem pH-Wert von 10 bis 11 verwendet wird.

4. Verfahren nach einem der vorhergehenden Ansprüche,
**dadurch gekennzeichnet,**
daß als Puffer Natrium- oder Kaliumcarbonat verwendet wird.

5. Verfahren nach einem der vorhergehenden Ansprüche,
**dadurch gekennzeichnet,**
daß der Puffer in einer Konzentration von 50 bis 500 mmol/l verwendet wird.

6. Verfahren nach einem der vorhergehenden Ansprüche,
**dadurch gekennzeichnet,**
daß als Detergens ein nichtionisches und/oder anionisches und/oder zwitterionisches Detergens verwendet wird.

**7.** Verfahren nach Anspruch 6,
**dadurch gekennzeichnet,**
daß als nichtionisches Detergens ein gerad- oder verzweigtkettiger Alkanol-Polyglykolether mit 8 bis 12 C-Atomen im Alkanolteil und durchschnittlich 4 bis 8 Glykoleinheiten pro Molekül verwendet wird.

**8.** Verfahren nach Anspruch 7,
**dadurch gekennzeichnet,**
daß der Alkanolpolyglykolether in einer Konzentration von 1 bis 5 Gew.-% verwendet wird.

**9.** Verfahren nach einem der vorhergehenden Ansprüche,
**dadurch gekennzeichnet,**
daß als anionisches Detergens ein Alkalisalz einer Gallensäure, insbesondere Natriumcholat, verwendet wird.

**10.** Verfahren nach Anspruch 6,
**dadurch gekennzeichnet,**
daß das anionische Detergens in einer Konzentraticn von 1 bis 10 mmol/l verwendet wird.

**11.** Verfahren nach einem der vorhergehenden Ansprüche,
**dadurch gekennzeichnet,**
daß zusätzlich ein kationisches Detergens verwendet wird.

**12.** Verfahren nach Anspruch 11,
**dadurch gekennzeichnet,**
daß als kationisches Detergens eine quartäre Ammonium-und/oder Pyridiniumverbindung verwendet wird.

**13.** Verfahren nach einem der vorhergehenden Ansprüche,
**dadurch gekennzeichnet,**
daß Uricase in einer Konzentration von 2 bis 10 U/ml verwendet wird.

**14.** Verfahren nach einem der vorhergehenden Ansprüche,
**dadurch gekennzeichnet,**
daß das peroxidatisch wirksame Enzym in einer Konzentration von 1 bis 5 U/ml verwendet wird.

**15.** Verfahren nach einem der vorhergehenden Ansprüche,
**dadurch gekennzeichnet,**
daß als peroxidatisch wirksames Enzym Peroxidase verwendet wird.

**16.** Verfahren nach einem der vorhergehenden Ansprüche,
**dadurch gekennzeichnet,**
daß das Tetrazoliumsalz in einer Konzentration von 0,1 bis 1 mmol/l, insbesondere 0,2 bis 0,6 mmol/l verwendet wird.

**17.** Verfahren nach einem der vorhergehenden Ansprüche,
**dadurch gekennzeichnet,**
daß als Tetrazoliumsalz-Verbindung Nitrotetrazoliumblau verwendet wird.

**18.** Verfahren nach einem der vorhergehenden Ansprüche,
**dadurch gekennzeichnet,**
daß die Messung 5 bis 15 Minuten nach Zugabe der Reagenzien erfolgt.

**19.** Verfahren nach einem der vorhergehenden Ansprüche,
**dadurch gekennzeichnet,**
daß die Messung bei einer Temperatur von 25 bis 37°C erfolgt.

**20.** Reagenz zur Bestimmung von Fructosamin in Körperflüssigkeiten, enthaltend ein Farbreagenz und oxidierend wirkende Enzyme,

**dadurch gekennzeichnet,**
daß es eine nicht reduzierend wirkende Puffersubstanz mit einem pH-Wert von 9 bis 12, eine Tetrazoliumsalzverbindung, Uricase sowie mindestens ein Detergens enthält.

**21.** Reagenz nach Anspruch 20,
**dadurch gekennzeichnet,**
daß es ein peroxidatisch wirksames Enzym enthält.

**22.** Reagenz nach Anspruch 21,
**dadurch gekennzeichnet,**
daß das peroxidatisch wirksame Enzym Peroxidase ist.

**Claims**

**1.** Process for the determination of fructosamine in body fluids by the reaction of the sample solution with a colour reagent and oxidising-acting enzymes,
characterised in that one mixes the sample liquid with a non-reducing-acting buffer solution with a pH value of 9 to 12, a tetrazolium salt compound and uricase, as well as at least one detergent, and kinetically measures the chronological change of the extinction in a temperature range between 20 and 40°C after a pre-incubation time of at least 5 minutes.

**2.** Process according to claim 1, characterised in that one adds a peroxidate-active enzyme.

**3.** Process according to claim 1 or 2, characterised in that a buffer solution with a pH value of 10 to 11 is used.

**4.** Process according to one of the preceding claims, characterised in that sodium or potassium carbonate is used as buffer.

**5.** Process according to one of the preceding claims, characterised in that the buffer is used in a concentration of 50 to 500 mMole/l.

**6.** Process according to one of the preceding claims, characterised in that a non-ionic and/or anionic and/or zwitterionic detergent is used as detergent.

**7.** Process according to claim 6, characterised in that a straight or branched-chained alkanol-polyglycol ether with 8 to 12 C-atoms in the alkanol part and, on average, 4 to 8 glycol units per molecule is used as non-ionic detergent.

**8.** Process according to claim 7, characterised in that the alkanol-polyglycol ether is used in a concentration of 1 to 5 wt.%.

**9.** Process according to one of the preceding claims, characterised in that an alkali metal salt of a bile acid, especially sodium cholate, is used as anionic detergent.

**10.** Process according to claim 6, characterised in that the anionic detergent is used in a concentration of 1 to 10 mMole/l.

**11.** Process according to one of the preceding claims, characterised in that a cationic detergent is additionally used.

**12.** Process according to claim 11, characterised in that a quaternary ammonium and/or pyridinium compound is used as cationic detergent.

**13.** Process according to one of the preceding claims, characterised in that uricase is used in a concentration of 2 to 10 U/ml.

**14.** Process according to one of the preceding claims, characterised in that the peroxidate-active enzyme is

EP 0 291 060 B1

used in a concentration of 1 to 5 U/ml.

15. Process according to one of the preceding claims, characterised in that peroxidase is used as peroxidateactive enzyme.

16. Process according to one of the preceding claims, characterised in that the tetrazolium salt is used in a concentration of 0.1 to 1 mMol/l, especially of 0.2 to 0.6 mMol/l.

17. Process according to one of the preceding claims, characterised in that nitrotetrazolium blue is used as tetrazolium salt compound.

18. Process according to one of the preceding claims, characterised in that the measurement takes place 5 to 15 minutes after addition of the reagents.

19. Process according to one of the preceding claims, characterised in that the measurement takes place at a temperature of 25 to 37°C.

20. Reagent for the determination of fructosamine in body fluids, containing a colour reagent and oxidising-acting enzymes, characterised in that it contains a non-reducing-acting buffer substance with a pH value of 9 to 12, a tetrazolium salt compound, uricase, as well as at least one detergent.

21. Reagent according to claim 20, characterised in that it contains a peroxidate-active enzyme.

22. Reagent according to claim 21, characterised in that the peroxidate-active enzyme is peroxidase.

**Revendications**

1. Procédé de détermination de la fructosamine dans les fluides corporels par réaction de la solution échantillon avec un réactif coloré et des enzymes oxydantes, caractérisé en ce que l'on additionne le liquide échantillon d'une solution tampon non réductrice d'un pH de 9 à 12, d'un composé d'un sel de tétrazolium et d'uricase, et d'au moins un détergent, et on mesure cinétiquement la modification temporelle de l'extinction dans un domaine de température compris entre 20 et 40°C après un temps de pré-incubation d'au moins 5 minutes.

2. Procédé selon la revendication 1, caractérisé en ce que l'on ajoute une enzyme peroxydante.

3. Procédé selon la revendication 1 ou 2, caractérisé en ce que l'on utilise une solution tampon d'un pH de 10 à 11.

4. Procédé selon l'une des revendications précédentes, caractérisé en ce que l'on utilise comme tampon le carbonate de sodium ou de potassium.

5. Procédé selon l'une des revendications précédentes, caractérisé en ce que l'on utilise le tampon en une concentration de 50 à 500 mmol/l.

6. Procédé selon l'une des revendications précédentes, caractérisé en ce que l'on utilise comme détergent un détergent non ionique et/ou anionique et/ou zwitterionique.

7. Procédé selon la revendication 6, caractérisé en ce que l'on utilise comme détergent non ionique un alcanolpolyglycoléther à chaîne linéaire ou ramifiée ayant 8 à 12 atomes de carbone dans la partie alcanol et en moyenne 4 à 8 unités de glycol par molécule.

8. Procédé selon la revendication 7, caractérisé en ce que l'on utilise l'alcanolpolyglycoléther en une concentration de 1 à 5% en poids.

9. Procédé selon l'une des revendications précédentes, caractérisé en ce que l'on utilise comme détergent anionique un sel alcalin d'un acide biliaire, en particulier le cholate de sodium.

10

**10.** Procédé selon la revendication 6, caractérisé en ce que l'on utilise le détergent anionique en une concentration de 1 à 10 mmol/l.

**11.** Procédé selon l'une des revendications précédentes, caractérisé en ce que l'on utilise en outre un détergent cationique.

**12.** Procédé selon la revendication 11, caractérisé en ce que l'on utilise comme détergent cationique un composé d'ammonium quaternaire et/ou de pyridinium quaternaire.

**13.** Procédé selon l'une des revendications précédentes, caractérisé en ce que l'on utilise l'uricase en une concentration de 2 à 10 U/ml.

**14.** Procédé selon l'une des revendications précédentes, caractérisé en ce que l'on utilise l'enzyme peroxydante en une concentration de 1 à 5 U/ml.

**15.** Procédé selon l'une des revendications précédentes, caractérisé en ce que l'on utilise comme enzyme peroxydante la peroxydase.

**16.** Procédé selon l'une des revendications précédentes, caractérisé en ce que l'on utilise le sel de tétrazolium en une concentration de 0,1 à 1 mmol/l, en particulier de 0,2 à 0,6 mmol/l.

**17.** Procédé selon l'une des revendications précédentes, caractérisé en ce que l'on utilise comme composé de sel de tétrazolium le bleu de nitrotétrazolium.

**18.** Procédé selon l'une des revendications précédentes, caractérisé en ce que la mesure a lieu 5 à 15 minutes après l'addition des réactifs.

**19.** Procédé selon l'une des revendications précédentes, caractérisé en ce que la mesure a lieu à une température de 25 à 37°C.

**20.** Réactif pour la détermination de la fructosamine dans les fluides corporels, contenant un réactif coloré et une enzyme oxydante, caractérisé en ce qu'il contient une substance tampon non réductrice d'un pH de 9 à 12, un composé d'un sel de tétrazolium, de l'uricase et au moins un détergent.

**21.** Réactif selon la revendication 20, caractérisé en ce qu'il contient une enzyme peroxydante.

**22.** Réactif selon la revendication 21, caractérisé en ce que l'enzyme peroxydante est la peroxydase.

# FIG.1

X = Normal-Serum

● = Diabetiker-Serum

# FIG.2

FIG.3